# EUROPÄISCHE PATENTSCHRIFT

(11) **EP 1 318 972 B1**
(45) Veröffentlichungstag und Bekanntmachung des Hinweises auf die Patenterteilung: **02.11.2006**
(21) Anmeldenummer: 01985250.8
(22) Anmeldetag: 11.09.2001
(51) Int. Cl.: C07C 45/74, C07C 47/21, C07C 29/17, C07C 31/125, C07C 1/22, C07C 49/647, C07C 13/19

(54) **VERFAHREN ZUR HERSTELLUNG VON VERZWEIGTEN ALKOHOLEN UND/ODER KOHLENWASSERSTOFFEN**
METHOD FOR PRODUCING BRANCHED ALCOHOLS AND/OR HYDROCARBONS
PROCEDE POUR PREPARER DES ALCOOLS ET/OU DES HYDROCARBURES RAMIFIES

(30) Priorität: 20.09.2000 DE 10046434
(43) Veröffentlichungstag der Anmeldung: 18.06.2003
(73) Patentinhaber: Cognis IP Management GmbH, 40589 Düsseldorf (DE)
(72) Erfinder: BOTH, Sabine, 40229 Düsseldorf (DE); SCHWERIN, Albrecht, 40217 Düsseldorf (DE); REUTER, Erich, 40591 Düsseldorf (DE); FIEG, Georg, 40822 Mettmann (DE); FALKOWSKI, Jürgen, 40789 Monheim (DE)
(86) Internationale Anmeldenummer: PCT/EP2001/010476
(87) Internationale Veröffentlichungsnummer: WO 2002/024621

(56) Entgegenhaltungen:
- DE-A- 3 133 078
- GB-A- 731 917
- US-A- 2 088 015
- US-A- 2 088 016
- US-A- 2 088 018
- US-A- 2 921 089

## Beschreibung

### Gebiet der Erfindung

Die Erfindung befindet sich auf dem Gebiet der kosmetischen Ölkörper und betrifft ein verbessertes Verfahren zur Herstellung beispielsweise von Guerbetalkoholen und Dialkylcydohexanen, welches in der Kondensation ohne Schwermetallkatalysatoren auskommt und sich bei verbesserter Selektivität durch eine höhere Reaktionsgeschwindigkeit auszeichnet.

### Stand der Technik

Guerbetalkohole stellen in 2-Position verzweigte primäre Alkohole dar, welche durch Kondensation von linearen Fettalkoholen erhalten werden. Die Produkte finden überwiegend als Ölkomponenten zur Herstellung von kosmetischen Emulsionen Verwendung. Zu ihrer Herstellung geht man in der Regel von Fettalkoholen aus, die zunächst unter dem Einfluß starker Basen und Schwermetallverbindungen, wie beispielsweise Kupfer oder Zinkoxid selbst kondensieren. Man nimmt an, dass unter den Reaktionsbedingungen der Alkohol zunächst zum Aldehyd dehydriert wird, dieser eine Aldolkondensation mit sich selbst eingeht und das Kondensationsprodukt anschließend zum Alkohol hydriert wird. Eine Übersicht hierzu findet sich beispielsweise in **Angew.Chem. 64, 212 (1952)**. In ähnlicher Weise erfolgt die Herstellung von Dialkylcyclohexanen durch zweifache Kondensation von Fettalkoholen mit Cyclohexanol in Gegenwart von Schwermetallen.

Von Nachteil ist jedoch, dass die Schwermetallkatalysatoren nach Beendigung der Reaktion wieder abgetrennt werden müssen, um gesetzliche Anforderungen zu erfüllen und sicherzustellen, dass sie nicht in der späteren Anwendung Irritationen hervorrufen. Die Abtrennung erfolgt in der Regel durch eine Wäsche mit anschließender Destillation, wobei diese mit nicht unerheblichen Produktverlusten verbunden ist. Ein weiterer Nachteil besteht in dem Umstand, dass die Reaktionszeiten sehr lang sind und die Selektivitäten zu wünschen übrig lassen.

### Beschreibung der Erfindung

Gegenstand der Erfindung ist ein Verfahren zur Herstellung von verzweigten Alkoholen und/oder Kohlenwasserstoffen, bei dem man Carbonylverbindungen in Gegenwart von Säuren oder Basen kondensiert und die resultierenden α,β-ungesättigten Kondensationsprodukte anschließend hydriert.

Überraschenderweise wurde gefunden, dass im Sinne des erfindungsgemäßen Verfahrens verzweigte Alkohole bzw. verzweigte Kohlenwasserstoffe auch ohne Mitverwendung von Schwermetallkatalysatoren in der Kondensation zugänglich sind, wobei sowohl verbesserte Selektivitäten als auch höhere Reaktionsgeschwindigkeiten erzielt werden.

### Carbonylverbindungen

Als Carbonylverbindungen kommen Aldehyde, Ketone sowie deren Gemische in Frage. Geeignete Aldehyde sind Fettaldehyde, die der Formel **(I)** folgen,

**R**^{**1**}**CHO** **(I)**

in der R¹ für einen linearen oder verzweigten Alkylrest mit 6 bis 12 und insbesondere 8 bis 10 Kohlenstoffatomen steht. Typische Beispiele sind Hexanol, Octanol, 2-Ethylhexanal, Decanol, Dodecanol sowie deren Gemische. Ebenfalls geeignet sind Fettketone, die der Formel **(II)** folgen,

**R**^{**2**}**COR**^{**3**} **(II)**

in der R² und R³ unabhängig voneinander für lineare oder verzweigte Alkylreste mit 6 bis 12 Kohlenstoffatomen steht. Typische Beispiele sind Dihexylketon, Dioctylketon, Di-2-Ethylhexylketon, Didecylketon oder Didodecylketon.

### Kondensation

Die Kondensationsreaktion kann in an sich bekannter Weise durchgeführt werden, d.h. man legt die Carbonylverbindungen zusammen mit den Säuren oder Basen vor und erhitzt sie auf Temperaturen im Bereich von 200 bis 250°C. Die Reaktion kann drucklos, oder bei Drücken bis 30, vorzugsweise bis 5 bar durchgeführt werden. Als Katalysatoren eignen sich insbesondere Alkalibasen, wie z.B. Alkalihydroxide oder Alkalicarbonate. Die Menge an Katalysatoren kann 1 bis 10, vorzugsweise 3 bis 5 Mol-% bezogen auf die Carbonylverbindungen betragen. Zur Verlagerung des Reaktionsgleichgewichtes auf die Produktseite empfiehlt sich in jedem Fall, das Kondensationswasser kontinuierlich abzudestillieren.

### Hydrierung

Die Hydrierung der als Zwischenprodukt anfallenden ungesättigten Aldehyde bzw. Ketone kann unter Einsatz üblicher Hydrierkatalysatoren, vorzugsweise auf Basis von Nickel-, Kupfer- und/oder Zink erfolgen. Üblicherweise wird die Hydrierung bei Temperaturen im Bereich von 20 bis 350, vorzugsweise 50 bis 250 °C und Drücken im Bereich von 1 bis 300, vorzugsweise 20 bis 250 bar durchgeführt. Anschließend können die Reaktionsprodukte durch Destillation aufgereinigt werden.

### Beispiele

**Beispiel 1**. In einer Rührapparatur bestehend aus Kolben, Heizpilz, Wasserabscheider, Rückflußkühler und Stickstoffüberleitung wurden 500 g (3,2 Mol) Decanal (99 Gew.-%ig) bei 20 °C mit 1 g (0,015 Mol) Kaliumhydroxid versetzt und auf 210 °C erhitzt. Das bei der Reaktion anfallende Wasser wurde kontinuierlich abdestilliert. Nach 3 h war die Reaktion beendet, das Reaktionsgemisch wurde auf 20 °C abgekühlt und das ausgefallene Kaliumhydroxid abfiltriert. Die resultierende klare Flüssigkeit enthielt 90 Gew.-% α,β-ungesättigten Aldehyd, 4 Gew.-% Trimere, 2 Gew.-% Ester und 4 Gew.-% nicht umgesetzten Ausgangsaldehyd. Das Reaktionsgemisch wurde in einen Autoklaven überführt und in Gegenwart eines Nickel-Katalysators bei 230 °C und 250 bar innerhalb von 3 h hydriert, bis keine Wasserstoffaufnahme mehr zu erkennen war. Das Hydrierprodukt bestand zu 90 Gew.-% aus 2-Octyldodecanol, 6 Gew.-% Decanol und 4 Gew.-% Trimeren. Nach Destillation wurde das 2-Octyldodecanol in einer Reinheit von 95,7 Gew.-% erhalten.

**Beispiel 2.** Analog Beispiel 1 wurden 500 g (3,9 Mol) Octanal in Gegenwart von 1,2 g (0,02 Mol) Kaliumhydroxid kondensiert. Das resultierende Produkt enthielt 88 Gew.-% α,β-ungesättigten Aldehyd, 6 Gew.-% Trimere, 2 Gew.-% Ester und 4 Gew.-% nicht umgesetztes Octanal. Nach Hydrierung wurde eine Mischung aus 88 Gew.-% 2-Hexyldecanol, 6 Gew.-% Octanol und 6 Gew.-% Trimeren erhalten. Nach Destillation wurde das 2-Hexyldecanol in einer Reinheit von 93,6 Gew.-% erhalten.

**Referenzbeispiel 3.** Analog Beispiel 1 wurden 650 g (5,0 Mol) 2-Ethylhexanal und 245 g (2,5 Mol) Cyclohexanon unter Zugabe von 40 g 45 Gew.-%iger wäßriger Kaliumhydroxidlösung kondensiert. Nach 2 h wurde die Reaktionstemperatur von 240 °C erreicht, wobei der Endpunkt durch die Beendigung der Wasserabscheidung angezeigt wurde. Das Produkt wurde mit heißem Wasser neutral gewaschen und mit Natriumsulfat getrocknet. Nach GC-Analyse lag eine Mischung aus 85,4 Gew.-% disubstituiertem Produkt, 8,2 Gew.-% monosubstituiertem Produkt, 1,3 Gew.-% 2-Ethylhexanal, 0,3 Gew.-% Cyclohexanon und 4,8 Gew.-% Polymeren vor. 500 g der Mischung wurden in Gegenwart von 14 g eines Nickel-Katalysators bei 245 °C und 20 bar über einen Zeitraum von 14 h hydriert, bis keine Wasserstoffaufnahme mehr erfolgte. Die naßchemische Analyse des Produktes ergab eine Säurezahl < 0,1, eine Iodzahl von 0,4 sowie eine Hydroxylzahl von 1. Die GC-Analyse lieferte als Zusammensetzung 85,4 Gew.-% 2,6-Di(2-ethylhexyl)cyclohexan, 8,2 Gew.-% 2(2-ethylhexyl)cyclohexan, 1,3 Gew.-% 2-Ethylhexan, 0,3 Gew.-% Cyclohexan und 4,8 Gew.-% Oligomere. Durch Destillation wurden die nicht umgesetzten Ausgangsstoffe abgetrennt.

## Patentansprüche

1. Verfahren zur Herstellung von verzweigten Alkoholen und/oder Kohlenwasserstoffen, bei dem man Carbonylverbindungen in Gegenwart von Säuren oder Basen kondensiert und die resultierenden α,β-ungesättigten Kondensationsprodukte anschließend hydriert, bei dem
man als Carbonylverbindungen Fettaldehyde der Formel **(I)** einsetzt,
**R**^{**1**}**CHO** **(I)**
in der R¹ für einen linearen oder verzweigten Alkylrest mit 6 bis 12 Kohlenstoffatomen steht, und/oder
man als Carbonylverbindungen Ketone der Formel **(II)** einsetzt,
**R**^{**2**}**COR**^{**3**} **(II)**
in der R² und R³ unabhängig voneinander für lineare oder verzweigte Alkylreste mit 6 bis 12 Kohlenstoffatomen steht, **dadurch gekennzeichnet, dass** man die Kondensation bei Temperaturen im Bereich von 200 bis 250 °C durchführt.

2. Verfahren nach Anspruch 1, **dadurch gekennzeichnet, dass** man als Carbonylverbindungen cyclische Ketone einsetzt.

3. Verfahren nach mindestens einem der Ansprüche 1 bis 2, **dadurch gekennzeichnet, dass** man die Hydrierung in Gegenwart von Nickel-, Kupfer- und/oder, Zink-Katalysatoren durchführt.

4. Verfahren nach mindestens einem der Ansprüche 1 bis 3, **dadurch gekennzeichnet, dass** man die Hydrierung bei Temperaturen im Bereich von 20 bis 350 °C durchführt

5. Verfahren nach mindestens einem der Ansprüche 1 bis 4, **dadurch gekennzeichnet, dass** man die Hydrierung bei Drücken im Bereich von 1 bis 300 bar durchführt.

6. Verfahren nach mindestens einem der Ansprüche 1 bis 5, **dadurch gekennzeichnet, dass** man die Hydrierprodukte durch Destillation aufreinigt.

7. Verfahren nach mindestens einem der Ansprüche 1 bis 6 **dadurch gekennzeichnet, dass** die Kondensation in Abwesenheit von Schwermetallen erfolgt.

## Claims

1. A process for the production of branched alcohols and/or hydrocarbons, in which carbonyl compounds are condensed in the presence of acids or bases and the resulting α,β-unsaturated condensation products are subsequently hydrogenated, fatty aldehydes corresponding to formula **(I)**:
**R**^{**1**}**CHO** **(I)**
in which R¹ is a linear or branched C₆₋₁₂ alkyl group,
and/or ketones corresponding to formula **(II):**
**R**^{**2**}**COR**^{**3**} **(II)**
in which R² and R³ independently of one another represent linear or branched C₆₋₁₂ alkyl groups,
being used as the carbonyl compounds, **characterized in that** the condensation is carried out at temperatures of 200 to 250°C.

2. A process as claimed in claim 1, **characterized in that** cyclic ketones are used as the carbonyl compounds.

3. A process as claimed in at least one of claims 1 to 2, **characterized in that** the hydrogenation is carried out in the presence of nickel, copper and/or zinc catalysts.

4. A process as claimed in at least one of claims 1 to 3, **characterized in that** the hydrogenation is carried out at temperatures of 20 to 350°C.

5. A process as claimed in at least one of claims 1 to 4, **characterized in that** the hydrogenation is carried out under pressures of 1 to 300 bar.

6. A process as claimed in at least one of claims 1 to 5, **characterized in that** the hydrogenation products are purified by distillation.

7. A process as claimed in at least one of claims 1 to 6, **characterized in that** the condensation is carried out in the absence of heavy metals.

## Revendications

1. Procédé pour préparer des alcools et/ou des hydrocarbures ramifiés, selon lequel on condense des composés carbonyle en présence d'acides ou de bases puis on hydrogène les produits de condensation α- et β-insaturés obtenus, et selon lequel on emploie comme composés carbonyle des aldéhydes gras de formule (I) :
R¹CHO (I)
dans laquelle R¹ représente un radical alkyle linéaire ou ramifié ayant 6 à 12 atomes de carbone, et/ou on emploie comme composés carbonyle des cétones de formule (II) :
R²COR³ (II)
dans laquelle R² et R³, représentent indépendamment l'un de l'autre, des radicaux alkyle linéaires ou ramifiés ayant 6 à 12 atomes de carbone,
**caractérisé en ce que**
l'on effectue la condensation à des températures dans la plage de 200 à 250 °C.

2. Procédé selon la revendication 1,
**caractérisé en ce que**
comme composés carbonyle on emploie des cétones cycliques.

3. Procédé selon au moins l'une des revendications 1 à 2,
**caractérisé en ce que**
l'on réalise l'hydrogénation en présence de catalyseurs au nickel, au cuivre, et/ou au zinc.

4. Procédé selon au moins l'une des revendications 1 à 3,
**caractérisé en ce qu'**
on réalise l'hydrogénation à des températures dans la plage de 20 à 350 °C.

5. Procédé selon au moins l'une des revendications 1 à 4,
**caractérisé en ce qu'**
on réalise l'hydrogénation à des pressions dans la plage de 1 à 300 bars.

6. Procédé selon au moins l'une des revendications 1 à 5,
**caractérisé en ce qu'**
on purifie les produits d'hydrogénation par distillation.

7. Procédé selon au moins l'une des revendications 1 à 6,
**caractérisé en ce que**
la condensation s'effectue en l'absence de métaux lourds.
